# EUROPÄISCHE PATENTSCHRIFT

(11) **EP 2 650 022 B1**
(45) Veröffentlichungstag und Bekanntmachung des Hinweises auf die Patenterteilung: **08.03.2017**
(21) Anmeldenummer: 13163129.3
(22) Anmeldetag: 10.04.2013
(51) Int. Cl.: A61L 2/08, B67B 3/00

(54) **Vorrichtung und Verfahren zum strahlungsbasierten Sterilisieren von Behältnisverschlüssen**
Method and device for radiation-based sterilisation of container closures
Dispositif et procédé destinés à la stérilisation à base de rayonnement de fermetures de récipients

(30) Priorität: 11.04.2012 DE 102012103116
(43) Veröffentlichungstag der Anmeldung: 16.10.2013
(73) Patentinhaber: Krones AG, 93073 Neutraubling (DE)
(72) Erfinder: Knott, Josef, 93073 Neutraubling (DE); Frankenberger, Günter, 93073 Neutraubling (DE); Sickert, Tino, 93073 Neutraubling (DE)
(74) Vertreter: Bittner, Bernhard

(56) Entgegenhaltungen:
- WO-A1-2009/009681
- WO-A1-2010/128532
- WO-A1-2012/000573
- WO-A1-2012/069101
- JP-A- 2001 171 624
- JP-A- 2002 128 030
- JP-A- 2008 195 428
- DATABASE WPI Week 201174 Thomson Scientific, London, GB; AN 2011-N36442 XP002699302, & JP 2011 213417 A (MITSUBISHI JUKOGYO KK) 27. Oktober 2011 (2011-10-27)

## Beschreibung

Die Erfindung bezieht sich auf eine Vorrichtung und ein Verfahren zum strahlungsbasierten Sterilisieren von Behältnisverschlüssen. Aus dem Stand der Technik sind diverse Verfahren zum Sterilisieren von Behältnisverschlüssen bekannt. Dabei ist es einerseits bekannt, dass die Behältnisverschlüsse mittels eines chemischen Mediums, wie beispielsweise Wasserstoffperoxid sterilisiert werden. In jüngerer Zeit wird jedoch teilweise gefordert, auf einen derartigen Chemikalieneinsatz zu verzichten.

Daher sind aus dem Stand der Technik auch Vorrichtungen und Verfahren bekannt, welche die Behältnisverschlüsse mittels Strahlung sterilisieren. Unter Strahlung wird im Folgenden insbesondere eine Ladungsträgerstrahlung verstanden, also beispielsweise eine Beaufschlagung mit Elektronen. Daneben kann es sich jedoch bei der Strahlung auch um andere Art von Strahlung handeln, wie beispielsweise ultraviolette Strahlung, radioaktive Strahlung, Röntgenstrahlung und dergleichen.

WO2009009681 offenbart einen Verschluss-Sterilisator mit mehreren, zueinander gewinkelten Strahlungsemittern, um eine umfängliche Behandlung der Verschlüsse zu gewährleisten. WO2012069101 und WO2012000573 offenbaren jeweils eine Vorrichtung zum Sterilisieren von Verschlüssen zum Verschliessen von Flaschen mit einem Transportsystem zum Bewegen der Verschlüsse durch wenigstens eine Behandlungszone, in der die Verschlüsse für das Sterilisieren oder Entkeimen mit einer UV-Strahlung beaufschlagt werden. WO2010128532 offenbart einen Elektronenstrahlsterilisator für Verschlüsse, wobei die Verschlüsse entlang der Behandlungsstrecke aktiv rollend gefördert werden. Ähnliche technische Lehren werden in JP2002128030, JP2011213417, JP2008195428 und JP2001171624 beschrieben.

Aus der WO 2010/128 532 A1 ist eine Einheit und ein Verfahren zum Sterilisieren von Behältnisverschlüssen bekannt. Dabei werden die Behältnisverschlüsse rollend entlang eines Transportpfades geführt und während der rollenden Bewegung mit Elektronenstrahlung bestrahlt.

Die beiden Elektronenstrahleinheiten sind dabei gegenüber bezüglich der zu sterilisierenden Behältnisverschlüsse angeordnet. Diese Vorrichtung erlaubt damit eine Sterilisierung einer inneren Bodenfläche der Verschlüsse und auch der dieser gegenüberliegenden äußeren Oberfläche. Damit kann hier diese innere Fläche sterilisiert werden, unabhängig davon, in welcher Orientierung der Verschluss durch die Vorrichtung rollt. Allerdings erlaubt diese Vorrichtung keine ausreichende Sterilisierung der Außenumfangsbereiche der Behältnisverschlüsse.

Die JP 2007 0 767 730 offenbart ebenfalls eine Sterilisationsvorrichtung zum Sterilisieren von Behältnisverschlüssen mittels Elektronenstrahlung. Auch dabei werden die Behältnisverschlüsse entlang eines vorgegebenen Pfades geführt, sodass sie durch die Strahlung hindurch gelangen. Bei dieser Vorrichtung ist es möglich, dass die Behältnisverschlüsse gedreht werden und von der Elektronenbestrahlungseinrichtung bestrahlt werden.

Die JP 2002 1 280 30 A beschreibt ebenfalls eine Vorrichtung zum Sterilisieren von Verschlüssen. Bei dieser Vorrichtung werden die Behältnisverschlüsse zwischen zwei Transportbändern gefördert, die sich mit unterschiedlichen Geschwindigkeiten bewegen können, sodass die Behältnisverschlüsse rotiert werden. Auch bei dieser Vorrichtung ist eine Sterilisation der Umfangsoberfläche der Behältnisverschlüsse nur schwer möglich.

Der vorliegenden Erfindung liegt daher die Aufgabe zugrunde, eine Vorrichtung und ein Verfahren zum Sterilisieren von Behältnisverschlüssen zur Verfügung zu stellen, welches sowohl eine Sterilisation der Umfangswandung der Verschlüsse als auch eine Sterilisation der Innenoberflächen dieser Behältnisverschlüsse erlaubt. Unter den Innenoberflächen werden im Folgenden diejenigen Bereiche der Verschlüsse verstanden, welche insbesondere auf die Mündung von Behältnissen aufgeschraubt werden.

Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Verschlusssterilisationsanordnung für Behältnisverschlüsse ist in Anspruch 1 definiert.

Erfindungsgemäß weist die Verschlusssterilisation eine Dreheinrichtung auf, welche die Behältnisverschlüsse wenigstens zeitweise während ihres Transportes mit der Transporteinrichtung um eine vorgegebene Drehachse dreht, wobei die Bestrahlungseinrichtung derart gegenüber dem Transportpfad der Behältnisverschlüsse angeordnet ist, dass die von der Bestrahlungseinrichtung ausgehende Strahlung sowohl auf eine äußere Umfangswandung der Behältnisverschlüsse trifft als auch auf einen Innenbereich der Behältnisverschlüsse. Erfindungsgemäß bestrahlt weiterhin die Bestrahlungseinrichtung die Behältnisverschlüsse aus zwei unterschiedlichen Richtungen. Zwar lässt sich, insbesondere bei einer Bestrahlung von Objekten mit Ladungsträgern wie insbesondere Elektronen keine genaue physikalische Strahlungsrichtung angeben. Unter der Bestrahlungsrichtung wird jedoch insbesondere diejenige Richtung verstanden, unter der die Ladungsträger im Mittel auf das zu sterilisierenden Behältnis treffen. Bevorzugt handelt es sich dabei um eine Richtung, welche im Wesentlichen senkrecht zu einem Austrittsfenster einer Ladungsträgererzeugungseinrichtung steht. Weiterhin handelt es sich bevorzugt bei der Strahlungsrichtung um eine geometrische Richtung, welche sich von der Strahlungseinrichtung zu den Behältnisverschlüssen erstreckt.

Zu diesem Zwecke kann die Bestrahlungseinrichtung mehrere Strahler bzw. Strahlungseinrichtungen aufweisen, die derart angeordnet sind, dass die Behältnisverschlüsse bevorzugt gleichzeitig aus mehreren Richtungen bestrahlt werden. Dabei ist ein Winkel zwischen diesen beiden Richtungen vorzugsweise größer als 30°, bevorzugt größer als 45°, bevorzugt größer als 60° und bevorzugt größer als 80°. Andererseits ist der besagte Winkel zwischen den Richtungen bevorzugt kleiner als 180°, bevorzugt kleiner als 150°, besonders bevorzugt kleiner als 120°und besonders bevorzugt kleiner als 100°. Der Winkel zwischen diesen beiden Richtungen ist dabei vorteilhaft in einer Ebene definiert, welche senkrecht zu dem Transportpfad der Behältnisverschlüsse steht. Vorteilhaft sind daher die beiden Richtungen auch nicht entgegengesetzt zueinander. Durch diese Bestrahlung kann sichergestellt werden, dass - insbesondere in Verbindung mit der oben beschriebenen Drehung der Behältnisverschlüsse, im Folgenden auch nur kurz als Verschlüsse bezeichnet - im Wesentlichen sämtliche Bereiche der Behältnisverschlüsse mit der besagten Bestrahlung beaufschlagt werden.

Es wird daher erfindungsgemäß vorgeschlagen, dass die Bestrahlung derart angeordnet wird, dass sowohl der Innenbereich der Behältnisverschlüsse sterilisiert wird, als auch die äußere Umfangswandung und besonders bevorzugt auch eine äußere Bodenfläche.

Vorteilhaft handelt es sich bei der Bestrahlungseinrichtung um einen Elektronenstrahler, das heißt die Bestrahlungseinrichtung weist eine Erzeugungseinrichtung für Elektronen auf sowie vorteilhaft auch eine Beschleunigungseinrichtung, welche die Elektronen in Richtung der Behältnisverschlüsse beschleunigt. Dabei kann diese Beschleunigungseinrichtung die Ladungsträger, insbesondere Elektronen, beispielsweise auf Energien im Bereich von 100 bis 200 keV beschleunigen.

Bei einer weiteren vorteilhaften Ausführungsform weist die Bestrahlungseinrichtung eine erste Bestrahlungseinrichtung auf, welche Strahlung unter einer ersten vorgegebenen Richtung auf die Behältnisverschlüsse richtet sowie eine zweite Strahlungseinrichtung, welche Strahlung unter einer zweiten vorgegebenen Richtung, welche sich von der ersten Richtung unterscheidet, auf die Behältnisverschlüsse richtet. Für diese Ausgestaltung sind daher zwei bevorzugt unabhängig voneinander steuerbare Strahlungseinrichtungen vorgesehen.

Insbesondere unterscheiden sich dabei die Richtungen der geometrischen Verbindungslinien zwischen den jeweiligen Strahlungseinrichtungen und den Verschlüssen.

Bei einer weiteren vorteilhaften Ausführungsform weist die Bestrahlungseinrichtung eine erste Strahlungseinrichtung auf, welche Strahlung unter einer ersten vorgegebenen Richtung auf die Behältnisverschlüsse richtet und eine Strahlungsumlenkeinrichtung, welche einen Teil der von der ersten Strahlungseinrichtung austretenden Strahlung auf die Behältnisverschlüsse umlenkt, insbesondere derart, dass die beiden Strahlungen unter den unterschiedlichen Richtungen auf die Behältnisverschlüsse treffen. Als derartige Strahlungseinrichtungen kommen dabei Reflektorbleche aus einem atomar dichten Material wie z.B. Gold (bzw. mit Goldbeschichtung) in Betracht.

Unter der oben erwähnten aktiven Transporteinrichtung wird daher verstanden, dass sich die Behältnisverschlüsse nicht nur entlang beispielsweise einer Transportrinne bewegen, sondern aktiv angetrieben werden. Bei weiteren aus dem Stand der Technik bekannten Verfahren befinden sich die Behältnisverschlüsse während der Bestrahlung in einer Verschlussrinne, die aufgrund ihrer Beschaffenheit sowohl die Transportrichtung der Verschlüsse vorgibt, als auch aufgrund ihrer Neigung für den Vortrieb sorgt. Derartige Rinnen sind dabei oftmals offen gestaltet, sodass möglichst viel Strahlung auf den Verschluss trifft.

Dennoch lassen sich in diesem Falle Beschattungseffekte nicht vermeiden, da der Verschluss weder kontrolliert gerollt noch aktiv bewegt wird. Zwar kann in kurzen Rinnenabschnitten die Rinne einseitig geöffnet werden, aber zur sicheren Führung des Verschlusses müssen zumeist Dreiviertel der Kontur der Verschlüsse umschlossen sein. Die Vereinzelung der Verschlüsse erfolgt im Stand der Technik beispielsweise mit einem Sternrad, sodass jeder Verschluss einzeln in die Kammer gleitet. Die Geschwindigkeit kann überwacht werden und ergibt sich aus der Zeitdifferenz zwischen dem Eintritt und dem Austritt. Allerdings ist eine aktive Beeinflussung der Geschwindigkeit nicht möglich.

Problematisch ist im Stand der Technik weiterhin die Beseitigung von Störungen, die aufgrund defekter, hängengebliebener Verschlüsse auftreten können. Wegen der entstehenden Röntgenstrahlung ist die Behandlungskammer umfassend abgeschirmt. Ein schneller Eingriff zur Störungsbehebung ist dabei oft nicht möglich. Daneben ist im Stand der Technik auch üblicherweise keine Ausschleusvorrichtung vorgesehen, die beispielsweise zum Ausschleusen fehlerhafter Verschlüsse vorteilhaft sein kann. Diese Nachteile werden durch die Verwendung von aktiven Transportsystemen und auch aktiven Vereinzelungs- und Beförderungs- und Bewegungssystemen der Behältnisverschlüsse innerhalb der Behandlungskammer zumindest weitgehend beseitigt.

Bei einer weiteren vorteilhaften Ausführungsform weist die Vorrichtung eine Vereinzelungseinrichtung auf, welche sicherstellt, dass die Behältnisverschlüsse vereinzelt in die Transporteinrichtung gelangen, das heißt derart, dass sich während des Transports der Behältnisverschlüsse diese nicht gegenseitig berühren.

Die oben erwähnte Dreheinrichtung, welche die Behältnisverschlüsse wenigstens zeitweise während ihres Transports mit der Transporteinrichtung um eine vorgegebene Drehachse dreht, kann ebenfalls in unterschiedlicher Weise ausgeführt werden. So wäre es beispielsweise möglich, dass die Behältnisverschlüsse entlang eines Transportpfades mittels einzelner Mitnehmer transportiert werden, wobei vorteilhaft diese Mitnehmer an einem besonders bevorzugt linearen Transportmittel wie einer Kette oder einem Riemen angeordnet sind. Dabei ist es möglich, dass die Behältnisverschlüsse über einen Eintaktstern auf Teilung gebracht und einzeln an diese Mitnehmer übergeben werden.

Vorteilhaft werden dabei die Behältnisverschlüsse auf einer waagerechten Bahn bzw. "hochkant" geschoben.

Bei einer weiteren vorteilhaften Ausführungsform ist die Transporteinrichtung daher derart gestaltet, dass sie die Behältnisverschlüsse wenigstens zeitweise während ihrer Sterilisation derart fördert, dass eine Symmetrieachse der Behältnisverschlüsse schräg bezüglich einer Einstrahlrichtung der sterilisierenden Strahlung steht. Dabei ist es sowohl möglich, dass die Behältnisverschlüsse an sich schräg gegenüber einer horizontalen Ebene gefördert werden. Es wäre jedoch auch möglich, dass die Behältnisverschlüsse selbst in einer horizontalen oder vertikalen Ebene gefördert werden und die Strahlungseinrichtung die Strahlung schräg zu diesen Ebenen einstrahlt.

Durch diese Schrägstellung wird erreicht, dass sich, insbesondere bei einer Drehbewegung der Behältnisverschlüsse sämtliche Innen- und Außenbereiche der Verschlüsse durch die Bestrahlungseinrichtung sterilisieren bzw. bestrahlen lassen.

Bei einer weiteren vorteilhaften Ausführungsform weist die Transporteinrichtung Stützkörper auf, welche die Behältnisverschlüsse während ihres Transports wenigstens teilweise an ihren Bodenflächen abstützen. So ist es beispielsweise möglich, dass die Behältnisverschlüsse einerseits an einem Bereich ihrer Umfangswandung und andererseits an ihren Bodenflächen abgestützt werden und anschließend von den Mitnehmern bewegt werden.

Bei einer weiteren vorteilhaften Ausführungsform weist die Transporteinrichtung eine erste Kontaktiereinrichtung auf, welche die Behältnisverschlüsse während deren Transport an einem Bereich ihrer Außenwandung, bevorzugt ihrer Umfangswandung kontaktiert. Weiterhin weist vorteilhaft die Transporteinrichtung eine zweite Kontaktiereinrichtung auf, welche die Behältnisverschlüsse während deren Transport an einem Bereich ihrer Außenwandung, bevorzugt ihrer Umfangswandung oder einer äußeren Bodenfläche kontaktiert. Es wäre auch möglich, dass beide Kontaktiereinrichtungen die Behältnisverschlüsse an ihrer äußeren Bodenfläche kontaktieren und eine Drehung der Behältnisverschlüsse beispielsweise durch eine Relativgeschwindigkeit zwischen diesen Kontaktiereinrichtungen erreicht wird.

Vorteilhaft werden die Verschlüsse von der Dreheinrichtung bzw. Kontaktelementen dieser Dreheinrichtung an zwei Flächen und insbesondere an zwei voneinander beabstandeten Flächen bzw. Bereichen (wobei es sich bei diesen Bereichen auch nahezu bzw. im Wesentlichen um Linien handeln kann) kontaktiert, die in einem von 0° oder 180° verschiedenen Winkel zueinander angeordnet sind. Vorteilhaft stehen die besagten Flächen bzw. Bereiche im Wesentlichen senkrecht zueinander. So wäre es möglich, dass eine Außenwand der Behältnisverschlüsse und eine Bodenfläche kontaktiert werden, oder auch zwei Bereiche der Außenwand, die im Wesentlichen senkrecht zueinander stehen. Vorteilhaft handelt es sich jedoch bei den kontaktierten Bereichen um äußere Wandungsbereiche der Behältnisverschlüsse, also insbesondere um solche Bereiche, welche nicht mit dem zu verschließenden Behältnis in Berührung kommen.

Falls zwei Bereiche der Umfangswandung des Behältnisverschlusses kontaktiert werden, stehen bevorzugt Tangenten an die Umfangswandung in diesen Bereichen zueinander in einem Winkel zwischen 30° und 150°, bevorzugt zwischen 60° und 120°, bevorzugt zwischen 80° und 100° und besonders bevorzugt in einem Winkel von ca. 90° zueinander.

Dabei unterscheidet sich vorteilhaft ein Reibungskoeffizient zwischen der ersten Kontaktiereinrichtung und der Außenoberfläche bzw. Umfangswandung der Behältnisverschlüsse von einem Reibungskoeffizienten zwischen der zweiten Kontaktiereinrichtung und der Außenoberfläche bzw. Umfangswandung der Behältnisverschlüsse. Auf diese Weise kann es zu einem kontrollierten Drehen der Behältnisverschlüsse um die vorgegebene Drehachse kommen. Vorteilhaft erstreckt sich diese Drehachse senkrecht zu einer Bodenfläche der Behältnisverschlüsse und besonders bevorzugt auch parallel zu den besagten Umfangswandungen.

So ist es beispielsweise möglich, dass eine Aufstandsfläche, das heißt die erste Kontaktierungseinrichtung eine gezahnte Oberfläche besitzt, die beispielsweise der Negativform der Riffelung der Verschlussaußenseite entspricht. Auf diese Weise verzahnt sich der Verschluss mit der Aufstandsfläche und muss definiert abrollen.

Durch die oben erwähnte Schrägstellung der Behältnisverschlüsse und deren Tendenz, nach hinten zu kippen, kann auch auf eine Führung an der offenen Vorderseite verzichtet werden und somit kann Strahlung ungehindert insbesondere in die Verschlussinnenseite gelangen. Weiterhin ist es möglich, die oben erwähnte Schrägstellung der Behältnisverschlüsse bzw. den Neigungswinkel zu variieren und damit unterschiedliche Bestrahlungswinkel zu erreichen. Bei dieser Ausführungsform werden daher die Behältnisverschlüsse nicht nur um die besagte Drehachse gedreht, sondern ihre Neigung entlang des Transportpfades wird ebenfalls geändert.

Damit werden auf diese Weise entlang des Transportpfades ebenso die oben erwähnten Strahlungsrichtungen der auf die Behältnisverschlüsse gerichteten Bestrahlung geändert. So kann beispielsweise eine senkrechte Bestrahlung dazu dienen, um die Tiefen der Dichtlippen der Behältnisverschlüsse zu erreichen und eine schräge Bestrahlung, um die Bereiche des Gewindes besser erreichen zu können. Weiterhin kann auch vorgesehen sein, dass die Behältnisverschlüsse nur entlang eines Abschnittes ihres Transportpfades bezüglich der erwähnten Drehachse gedreht werden und in anderen Bereichen lediglich transportiert werden.

Vorteilhaft weist daher die Vorrichtung eine Neigungsänderungseinrichtung auf, welche eine Neigung der Behältnisverschlüsse gegenüber deren Transportpfad verändert.

Wie oben erwähnt, ist es möglich, dass zwei Emitter bzw. Strahlungseinrichtungen vorgesehen sind, wobei einer für die Verschlussinnenseite und einer für die Verschlussaußenseite dient. Dabei sind diese Strahlungseinrichtungen im Gegensatz zu bekannten Systemen vorteilhaft nicht einander gegenüber, sondern in einem hiervon abweichenden Winkel, der vorteilhaft zwischen 30° und 150°, vorteilhaft zwischen 60° und 120°, vorteilhaft zwischen 75° und 105° und besonders bevorzugt bei ca. 90° liegt, zueinander angeordnet.

Bei der oben erwähnten Strahlungsumlenkeinrichtung kann es sich beispielsweise um ein Streublech oder ein Reflektor handeln, welches bewirkt, dass die neben dem Verschluss vorbeigehende Strahlung aus dem Emitter der Innenbehandlung so gestreut und reflektiert wird, dass damit die Verschlussrückseite sterilisiert werden kann.

Weiterhin ist es auch möglich, dass die Sterilisationseinrichtung eine Erfassungseinrichtung zu sogenannten Strahlungsaussetzern aufweist. Insbesondere bei Elektronenbestrahlungen kann es infolge von sogenannten Arcs dazu kommen, dass die Elektronenstrahlung kurzzeitig aussetzt. In diesem Falle wird auch die Sterilisation unterbunden. Hier ist es möglich, dass eine Steuerungseinrichtung die Transporteinrichtung derart steuert, dass der Transport der Behältnisverschlüsse bei Auftreten derartiger Aussetzer angehalten wird. Auf diese Weise kann verhindert werden, dass Behältnisverschlüsse unbehandelt bleiben oder weniger lang behandelt werden als vorgesehen. Sollte weiterhin während des Wiederhochfahrens der Strahlungseinrichtung eine Überbehandlung der Behältnisverschlüsse drohen, wäre es auch möglich, dass die Transporteinrichtung die Behältnisverschlüsse entgegen des Transportpfades, also rückwärts, transportiert, um beispielsweise die Behältnisverschlüsse aus der Transporteinrichtung zu entleeren.

Bei einer weiteren vorteilhaften Ausführungsform weist die Vorrichtung eine Auswerfeinrichtung auf, mit der einzelne Behältnisverschlüsse aus dem Transportpfad ausgeschleust werden können. So können beispielsweise fehlerhafte Behältnisverschlüsse ausgeschleust werden oder auch Behältnisverschlüsse, welche mit einer zu niedrigen oder zu hohen Strahlungsdosis beaufschlagt wurden.

Bei der oben erwähnten zweiten Kontaktierungseinrichtung kann es sich beispielsweise um einen beweglichen Schieber oder Mitnehmer handeln und bei der ersten Kontaktierungseinrichtung beispielsweise um eine stationäre Platte. Vorteilhaft ist daher eine der beiden Kontaktierungseinrichtungen stationär und besonders bevorzugt die andere beweglich angeordnet. Dadurch lässt sich mit einer Steuerung für die Antriebseinrichtung die Behandlungsgeschwindigkeit für die Behältnisverschlüsse beeinflussen bzw. ändern.

Es wäre jedoch auch möglich, dass die Behältnisverschlüsse wenigstens zeitweise mittels zwei Transportbändern transportiert werden, die sich mit unterschiedlicher Geschwindigkeit bewegen.

Die vorliegende Erfindung ist weiterhin auf ein Verfahren zum Sterilisieren von Behältnisverschlüssen nach Anspruch 9 gerichtet.
zumindest zeitweise während dieses Transports nicht gegenseitig berühren. Dabei werden die Behältnisverschlüsse wenigstens zeitweise während dieses Transports mittels wenigstens einer Bestrahlungseinrichtung mit einer sterilisierenden Bestrahlung bestrahlt. Erfindungsgemäß werden die Behältnisverschlüsse wenigstens zeitweise während ihrer Sterilisation um eine vorgegebene Drehachse gedreht und dabei wird sowohl eine Umfangswandung, insbesondere eine äußere Umfangswandung der Behältnisverschlüsse, als auch ein Innenbereich der Behältnisverschlüsse von der Bestrahlungseinrichtung bestrahlt. Insbesondere werden die Behältnisverschlüsse mit Ladungsträgern bestrahlt, also genauer mit den Ladungsträgern beaufschlagt. Bei diesen Ladungsträgern handelt es sich insbesondere um Elektronen.

Bei einem weiteren vorteilhaften Verfahren wird auch ein Bodenbereich der Behältnisverschlüsse wenigstens zeitweise bestrahlt.

Vorteilhaft werden die Behältnisverschlüsse wenigstens zeitweise während ihrer Sterilisation durch einen Reinraum gefördert. Dieser Reinraum kann dabei beispielsweise mit Sterilluft beaufschlagt sein und ist vorteilhaft gegenüber einer Umgebung abgetrennt. Besonders vorteilhaft ist der Reinraum mit einem sterilen Inertgas wie etwa Stickstoff oder Helium beaufschlagt, um so die Wechselwirkungen der Elektronen mit den Gasmolekülen so gering wie möglich zu halten.

Vorteilhaft werden die Behältnisverschlüsse aus wenigstens zwei unterschiedlichen Strahlungsrichtungen bestrahlt. Vorteilhaft handelt es sich bei den Behältnisverschlüssen um Kunststoffverschlüsse. Diese eignen sich in besonderer Weise für eine Beaufschlagung mit Ladungsträgern.

Bei einem weiteren vorteilhaften Verfahren werden die Behältnisverschlüsse insbesondere vor der Sterilisierung vereinzelt.

Weitere Vorteile und Ausführungsformen ergeben sich aus den beigefügten Figuren. Darin zeigen:
- Fig. 1: eine teilweise Darstellung einer erfindungsgemäßen Vorrichtung;
- Fig. 2: eine teilweise Darstellung einer erfindungsgemäßen Vorrichtung in einer weiteren Ausführungsform;
- Fig. 3: eine teilweise Darstellung einer erfindungsgemäßen Vorrichtung in einer weiteren Ausführungsform;
- Fig. 4: eine weitere Teilansicht einer erfindungsgemäßen Vorrichtung;
- Fig. 5: eine schematische Darstellung zur Veranschaulichung der Sterilisationsvorgänge;
- Fig. 6: eine Ansicht auf eine erfindungsgemäße Vorrichtung entlang des Transportpfades der Verschlüsse;
- Fig. 7: eine Ansicht einer weiteren Ausführungsform einer erfindungsgemäßen Vorrichtung;
- Fig. 8: eine weitere Ansicht einer erfindungsgemäßen Vorrichtung;
- Fig. 9: eine weitere Ansicht einer erfindungsgemäßen Vorrichtung;
- Fig. 10a, 10b: zwei Ansichten einer weiteren Ausführungsform einer erfindungsgemäßen Vorrichtung;
- Fig. 11a - 11d: vier schematische Darstellungen zur Veranschaulichung einer erfindungsgemäßen Vorrichtung; und
- Fig. 12a - 12c: drei schematische Darstellungen zur Veranschaulichung der Erfindung.

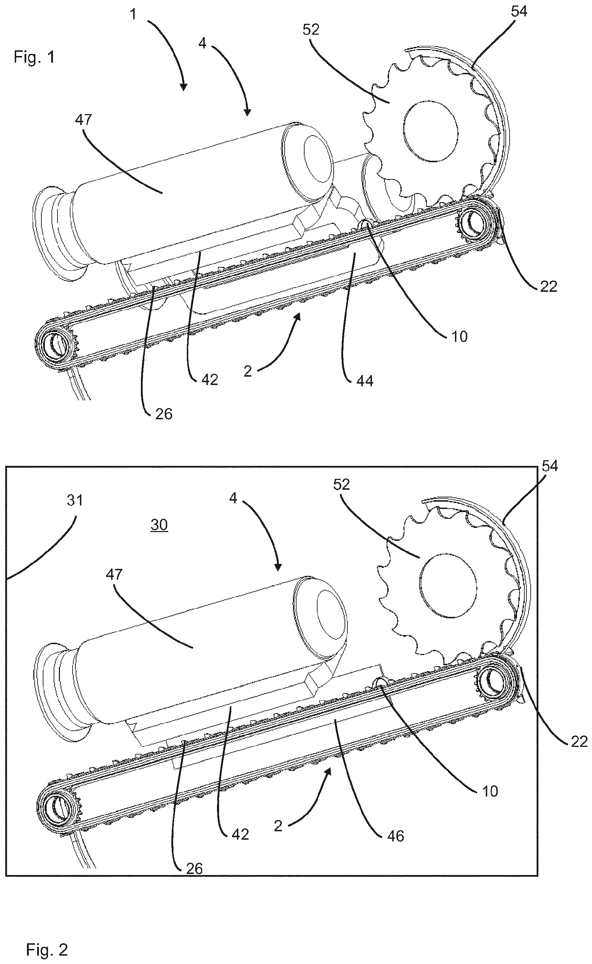

Fig. 1 zeigt eine erfindungsgemäße Vorrichtung 1 in einer ersten Ausführungsform. Dabei ist eine in ihrer Gesamtheit mit 2 bezeichnete Transporteinrichtung vorgesehen, welche Behältnisverschlüsse 10 transportiert. Dabei werden bei dieser Ausführungsform die Behältnisverschlüsse 10 im Wesentlichen hochkant auf einer waagerechten Bahn geschoben. Das Bezugszeichen 52 kennzeichnet ein Eintaktrad, welches eine vereinzelte Zuführung der Behältnisverschlüsse an die Transporteinrichtung 2 ermöglicht. Dabei werden die Behältnisverschlüsse 10 gegenüber einer stationär angeordneten Schiene 54 verschoben.

Das Bezugszeichen 22 bezieht sich auf ein hier umlaufendes Transportband, an dem eine Vielzahl von zweiten Kontakteinrichtungen 26 hier fest angeordnet ist. Damit bewegen sich die zweiten Kontaktiereinrichtungen mit dem Transportband 22 mit und damit auch teilweise in Richtung des Transportpfades der Behältnisverschlüsse 10.

Das Bezugszeichen 42 bezieht sich auf eine erste Strahlungseinrichtung, welche die Behältnisverschlüsse mit Elektronen bestrahlt. Das Bezugszeichen 44 kennzeichnet eine entsprechende zweite Strahlungseinrichtung, welche die Behältnisverschlüsse bestrahlt. Das Bezugszeichen 47 kennzeichnet eine Elektronenerzeugungseinrichtung, welche die Elektronen, welche zur Bestrahlung der Behältnisverschlüsse 10 dienen, erzeugt. Damit werden bei dieser Ausführungsform die Behältnisverschlüsse aus zwei Richtungen mit Elektronen bestrahlt, nämlich durch die erste Strahlungseinrichtung 42 und durch die zweite Strahlungseinrichtung 44. Diese beiden Strahlungseinrichtungen bilden zusammen die Bestrahlungseinrichtung 4 zum Bestrahlen der Behältnisverschlüsse.

Die Bestrahlungseinrichtung weist dabei vorteilhaft auch eine Ladungsträgererzeugungseinrichtung sowie eine Ladungsträgerbeschleunigungseinrichtung auf, welche die so erzeugten Ladungsträger, bei denen es sich insbesondere um Elektronen handelt, beschleunigt.

Fig. 2 zeigt eine erfindungsgemäße Vorrichtung in einer weiteren Ausführungsform. Dabei ist wiederum das oben erwähnte Eintaktrad 52 vorgesehen. Der wesentliche Unterschied besteht hier darin, dass in diesem Fall lediglich eine Strahlungseinrichtung 42 vorgesehen ist, zusätzlich jedoch ein Reflektorelement, bzw. eine Strahlungsumlenkeinrichtung 46, welches zumindest einen Teil der von der ersten Strahlungseinrichtung 42 kommenden Strahlung reflektiert und wiederum auf die Behältnisverschlüsse 10 richtet. Damit werden auch bei dieser Ausführungsform die Behältnisverschlüsse aus zwei Richtungen bestrahlt.

Vorteilhaft richtet dabei das Reflektorelement die Strahlung auf eine Außenseite bzw. Rückseite der Behältnisverschlüsse, welche im Rahmen einer Sterilisierung weniger kritisch ist als die Verschlussinnenseite, d.h. diejenige Seite, die unmittelbar mit dem zu verschließenden Behältnis in Berührung kommt.

Das Bezugszeichen 30 kennzeichnet grob schematisch einen Reinraum, innerhalb dessen die Behältnisverschlüsse sterilisiert werden. Dieser Reinraum kann dabei durch ein Gehäuse 31 gegenüber einer (unsterilen) Umgebung abgegrenzt werden. Dabei wäre es auch denkbar, dass dieser Reinraum 30 kleiner gewählt ist, als in den Figuren gezeigt und beispielsweise kanalartig die Transporteinrichtung 2 umgibt, wobei vorteilhaft die Strahlungserzeugungseinrichtungen außerhalb des Reinraums angeordnet sind, und/oder bevorzugt die Strahlungseinrichtungen 42 bzw. 44 bevorzugt einen Teil des Gehäuses 31 bilden. Vorteilhaft erstreckt sich jedoch dieser Reinraum auch noch in der Bewegungsrichtung der Behältnisverschlüsse 10 stromabwärts bezüglich der Sterilisationsvorrichtung 1 und schließt direkt an einen Reinraum an, in welchem eine Verschließeinrichtung zum Verschließen von Behältnissen mit den sterilisierten Behältnisverschlüssen angeordnet ist. Die Behältnisverschlüsse können dem Reinraum 30 beispielsweise über eine Schleuseneinrichtung zugeführt werden. Vorteilhaft ist innerhalb des Reinraums 30 ein steriles gasförmiges Medium (beispielsweise Sterilluft), insbesondere unter einem gegenüber dem Außendruck leicht erhöhten Druck angeordnet.

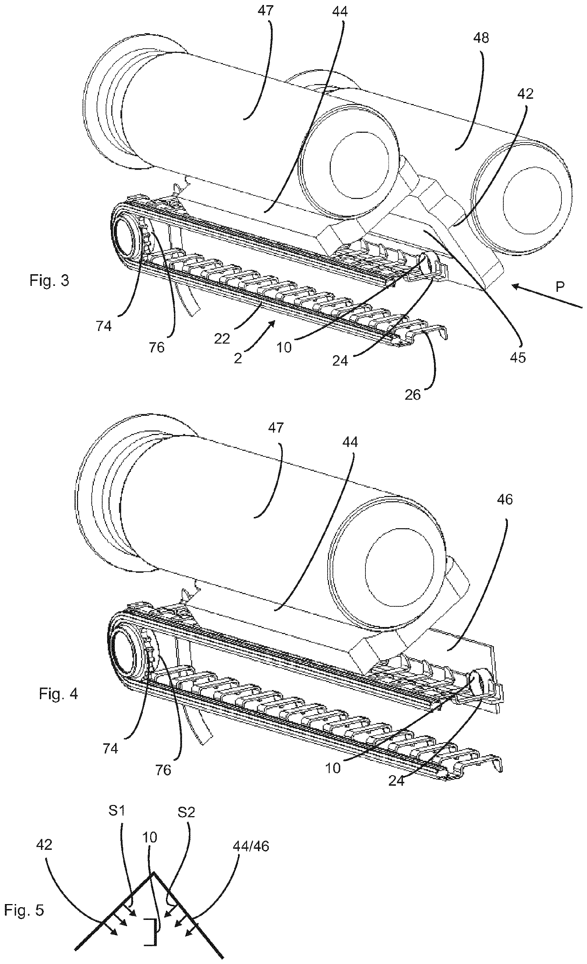

Fig. 3 zeigt eine weitere Ausführungsform einer erfindungsgemäßen Vorrichtung. Bei dieser Ausführungsform sind wiederum zwei Strahlungseinrichtungen 42 und 44 vorgesehen, zusätzlich jedoch auch zwei getrennte bzw. unabhängige Strahlungserzeugungseinrichtungen 47, 48. Auch ist in Fig. 3 die Ausführung der Transporteinrichtung 2 genauer dargestellt. Man erkennt hier wiederum die ersten Kontakteinrichtungen 26, welche hier die Behältnisse an ihren Seitenrändern kontaktieren. Daneben ist eine zweite Kontaktiereinrichtung 24 von einer feststehenden bzw. stationär angeordneten Schiene, der gegenüber die Behältnisverschlüsse abrollen, vorgesehen.

Dabei ist ein Reibungskoeffizient zwischen der Schiene 24 und den Behältnisverschlüssen 10 größer als ein Reibungswiderstand zwischen den zweiten Kontaktiereinrichtungen 26 bzw. den Mitnehmern 26 und den Behältnisverschlüssen. Auf diese Weise wird bewirkt, dass die Behältnisverschlüsse nicht nur entlang des Transportpfades P verschoben werden, sondern auch um eine senkrecht zu einer Deckelfläche der Behältnisverschlüsse stehende Drehachse gedreht werden. Auf diese Weise ist eine vollumfängliche Bestrahlung der Verschlüsse 10 möglich.

Das Bezugszeichen 74 bezieht sich auf ein Antriebszahnrad zum Antreiben eines Transportbandes 22 und das Bezugszeichen 76 auf eine Antriebseinrichtung wie einen Motor. Das Bezugszeichen 45 kennzeichnet ein Austrittsfenster, durch welches die in der Strahlungserzeugungseinrichtung 48 erzeugte Elektronenstrahlung austreten kann. Dabei kann es sich beispielsweise um ein Titanfenster handeln. Es wird an dieser Stelle draufhingewiesen, dass es sich bei der Strahlung zwar hier um Elektronen handelt, es wäre jedoch auch möglich, dass als Strahlung eine andere Art von Strahlung, wie beispielsweise UV-Strahlungen oder Röntgenstrahlungen oder dergleichen verwendet wird. Das Bezugszeichen P kennzeichnet den Transportpfad der Behältnisverschlüsse.

Fig. 4 zeigt eine weitere Ausführungsform der erfindungsgemäßen Vorrichtung. Bei dieser Ausführungsform ist keine zweite Strahlungseinrichtung 42 vorgesehen, dafür wiederum das oben erwähnte Reflektorelement 46, welches von der Strahlungseinrichtung 44 ausgehende Strahlung auf die Behältnisverschlüsse umlenkt.

Fig. 5 veranschaulicht die Bestrahlung der Behältnisverschlüsse. Man erkennt, dass die Behältnisverschlüsse in zwei Richtungen (Strahlungsrichtung S1 und Strahlungsrichtung S2) mit Strahlung beaufschlagt werden. Dabei stehen bei dieser Ausführungsform die beiden Strahlungsrichtungen S1 und S2 im Wesentlichen senkrecht zueinander. Zusätzlich dazu wird, wie oben erwähnt, der Behältnisverschluss 10 gedreht, sodass insgesamt eine vollumfängliche Bestrahlung der Behältnisverschlüsse während des Transports entlang des Transportpfades P möglich ist.

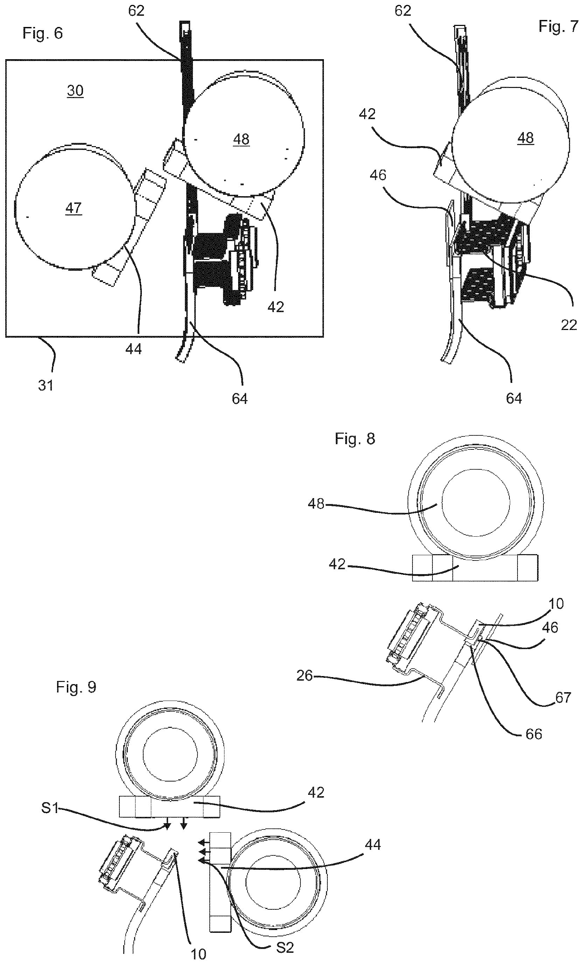

Fig. 6 zeigt eine weitere Darstellung einer erfindungsgemäßen Vorrichtung. Auch hier sind zwei Strahlungseinrichtungen 42 und 44 vorgesehen. Über eine Zuführeinrichtung 62, wie beispielsweise eine Zuführrinne, werden die Behältnisverschlüsse der Vorrichtung zugeführt und über eine Abführeinrichtung, bei der es sich ebenfalls um eine Rinne 64 handeln kann, werden die Verschlüsse wieder abgeführt.

Fig. 7 zeigt eine weitere Ausgestaltung, wobei auch bei dieser Ausführungsform nur eine Strahlungseinrichtung 42 vorgesehen ist sowie ein Reflektorelement 46, welches die Strahlung, insbesondere Strahlung, welche die Verschlüsse nicht unmittelbar trifft, auf die Behältnisverschlüsse 10 umlenkt. Dazu erkennt man hier wieder das Transportband 22.

Fig. 8 zeigt eine schematische Darstellung der erfindungsgemäßen Vorrichtung. Während bei Figur 7 die Strahlungseinrichtung 42 schräggestellt ist und die Behältnisverschlüsse vertikal transportiert werden, ist bei der in Figur 8 gezeigten Ausführungsform die Strahlungseinrichtung 42 horizontal ausgerichtet, das heißt die Strahlrichtung S1 verläuft hier zunächst im Wesentlichen in vertikaler Richtung. Ein Teil der Strahlung wird wiederum über das Reflektorelement 46 umgelenkt (S2). Das Bezugszeichen 66 kennzeichnet eine (stationär angeordnete) Halteeinrichtung, welche wiederum eine Transportfläche 67, der gegenüber die Behältnisverschlüsse gleiten, hält. Diese Transportfläche 67 ist dabei mit einer Vielzahl von (nicht gezeigten) Öffnungen versehen, durch welche hindurch die Verschlüsse bestrahlt werden können. Damit ist auch bei der in Figur 8 gezeigten Ausführungsform eine vollumfängliche Bestrahlung der Behältnisverschlüsse 10 möglich.

Bei der in Fig. 9 gezeigten Ausführungsform sind wiederum zwei Strahlungseinrichtungen 42, 44 vorgesehen, die hier die Behältnisverschlüsse unter zueinander senkrechten Winkeln bestrahlen. Es wird jedoch darauf hingewiesen, dass die Strahlungsrichtung S1 und S2 nicht notwendigerweise senkrecht stehen müssen, sondern auch davon abweichende Winkel, beispielsweise zwischen 60° und 120°, aufweisen können.

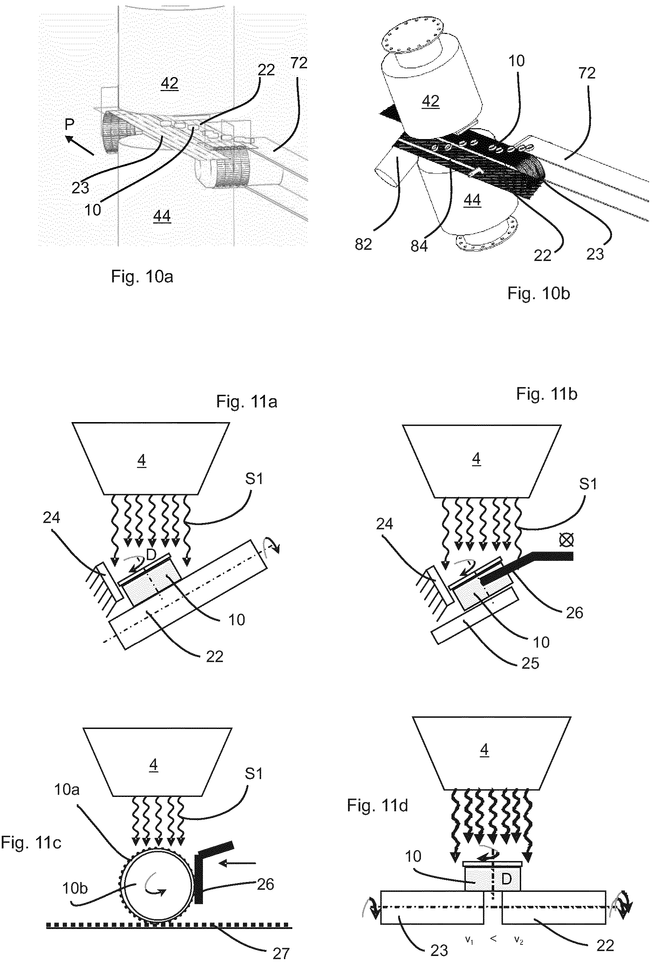

Die Fig. 10a und 10b zeigen eine weitere Ausgestaltung einer erfindungsgemäßen Vorrichtung. Bei der in Fig. 10a gezeigten Ausführungsform ist wiederum ein erstes Transportband 22 vorgesehen sowie auch ein zweites Transportband 23, welches die Behältnisverschlüsse entlang des Transportpfades P fördert. Bei dieser Ausführungsform sind zwei Strahlungseinrichtungen 42 und 44 entgegengesetzt ausgerichtet, um die Behältnisverschlüsse 10 von allen Seiten gleichmäßig bestrahlen zu können. Das Transportband 22 bzw. auch das Transportband 23, auf dem die Verschlüsse mit geringem Abstand zueinander befördert werden, verläuft hier horizontal. Weiterhin weist das Transportband eine Vielzahl von Öffnungen auf, ist beispielsweise als weitmaschiges Transportband ausgestaltet, damit die Verschlüsse auch von unten her bestrahlt werden können. Auf der Oberseite sind die Behältnisverschlüsse 10 keinerlei Beschattung ausgesetzt, das heißt, dass die Behältnisverschlüsse 10 hier jeweils derart auf den Transportbändern 22, 23 liegen, dass ihre Öffnungen nach oben, das heißt in Richtung der Strahlungseinrichtung 42, weisen.

Um auch die Beschattung auf der Rückseite der Behältnisverschlüsse zu minimieren, ist wie oben erwähnt das Transportband, bzw. die Transportkette bevorzugt als Edelstahlmattenkette mit möglichst großer Siebweite bzw. Maschenweite ausgeführt. Daneben wäre es auch denkbar, die Behältnisverschlüsse 10 während des Transports zu drehen, beispielsweise indem sich die beiden Transportbänder 22 und 23 mit unterschiedlicher Geschwindigkeit bewegen.

Fig. 10b zeigt eine weitere Ausführungsform, wobei hier zusätzlich das Transportband 22 anders geneigt ist, als das Transportband 23. Man erkennt hier, dass eine Antriebseinrichtung 82, wie beispielsweise eine Umlenkrolle, welche das Transportband 22 antreibt, geneigt ist. Damit trifft auch hier die Strahlung wieder schräg auf die einzelnen Behältnisverschlüsse 10. Das Bezugszeichen 84 kennzeichnet einen Transportsteg, der die Behältnisverschlüsse stützt, sodass sie nicht nach unten herabfallen. Auch kann durch diesen Steg, der hier auch als die oben beschriebene Kontaktiereinrichtung wirkt, wie unten veranschaulicht wird, eine Drehung der Behältnisverschlüsse erreicht werden. Auf diese Weise ist auch eine vollumfängliche Bestrahlung der Behältnisverschlüsse möglich.

Vorteilhaft liegt der Neigungswinkel des zweiten Transportbandes 22 gegenüber dem ersten Transportband 23 in einem Bereich zwischen 10° und 60°, bevorzugt zwischen 10° und 40°, bevorzugt zwischen 10° und 30°. Das Bezugszeichen 72 bezieht sich auf ein Zuführband.

Die Fig. 11a - 11d zeigen unterschiedliche Ausführungsformen, um eine Drehung der Behältnisverschlüsse 10 zu erreichen. Bei der in Fig. 11 a gezeigten Ausführungsform, welche der in Fig. 10b gezeigten Ausführungsform ähnelt, ist eine erste Kontaktiereinrichtung 24 vorgesehen, die hier stationär angeordnet ist und gegenüber der die Behältnisverschlüsse 10 abrollen. Dabei werden die Behältnisverschlüsse 10 mittels des Transportbandes 22 gefördert und drehen sich, wie veranschaulicht, um ihre Drehachse D.

Bei der in Fig. 11b gezeigten Ausführungsform ist auch eine Stützfläche 25 für die Behältnisverschlüsse vorgesehen, welche auch stationär angeordnet sein kann. Daneben sind zweite Kontaktiereinrichtungen 26 vorgesehen, welche die Behältnisverschlüsse 10 schieben und damit auch drehen.

Fig. 11c zeigt eine weitere Ausführungsform zum Transportieren der Behältnisverschlüsse. Dabei ist wiederum eine Außenwandung 10a der Behältnisverschlüsse 10 dargestellt sowie auch eine Innenfläche bzw. ein Innenbereich 10b. Hier werden die Behältnisverschlüsse 10 auf einer waagerechten Bahn hochkant geschoben, das heißt, eine Aufstandsfläche weist eine Riffelung 27 auf, welche hier mit einer Umfangsriffelung des Behältnisverschlusses 10 ineinandergreift. Dabei verzahnt sich der Behältnisverschluss 10 mit der Aufstandsfläche und kann so definiert abrollen. Wie oben erwähnt kann hier zusätzlich der Behältnisverschluss schräggestellt werden, um eine Strahlung schräg in den Verschluss hinein zu erreichen.

Auch kann durch die Schrägstellung des Verschlusses dessen Tendenz, nach hinten kippen zu wollen, entgegengewirkt werden. Daneben wäre es auch möglich, dass die Behältnisverschlüsse leicht aufwärtsgefördert werden, sodass auf eine Führung von vorne verzichtet werden kann und so die Strahlung ungehindert die Verschlussinnenseite erreichen kann. Weiterhin wäre es möglich, während des Transportes den Neigungswinkel des Verschlusses zu variieren, beispielsweise von 60° bis 0°, das heißt waagerecht, um damit verschiedene Bestrahlungswinkel zu erreichen zum Beispiel eine senkrechte Bestrahlung, um die Tiefen der Dichtlippen zu erreichen sowie auch eine Schrägstellung, um die Flächen des (Innen)Gewindes besser erreichen zu können.

Fig. 11d zeigt eine weitere Ausführungsform, welche ebenfalls eine Drehung der Behältnisverschlüsse 10 bewirkt. Hier wird der Verschluss 10 auf zwei Transportbändern 22, 23 bewegt, die sich mit unterschiedlichen Geschwindigkeiten bewegen. Auf diese Weise kommt es auch zu einer Drehung des Behältnisverschlusses 10, um seine Drehachse D.

Daneben ist es auch möglich, dass eine Vereinzelung der Verschlüsse mittels zwei unterschiedlich schnell laufenden Bändern realisiert wird. So kann ein langsameres Zuführband vorgesehen werden sowie ein schneller laufendes weiterführendes Band, sodass ein Abstand zwischen den Behältnisverschlüssen erhöht wird.

Durch die erfindungsgemäße Vorgehensweise wird ein von der Verschlussform unabhängiger Transport der Behältnisverschlüsse 10 erreicht. Auch ist im Gegensatz zum Stand der Technik die Geschwindigkeit exakt steuer- und kontrollierbar. Daneben können auch Verschattungseffekte bzw. Beschattungseffekte auf der Oberseite des Behältnisverschlusses vollständig verhindert werden und auch die Beschattungseffekte auf der Unterseite eingeschränkt werden.

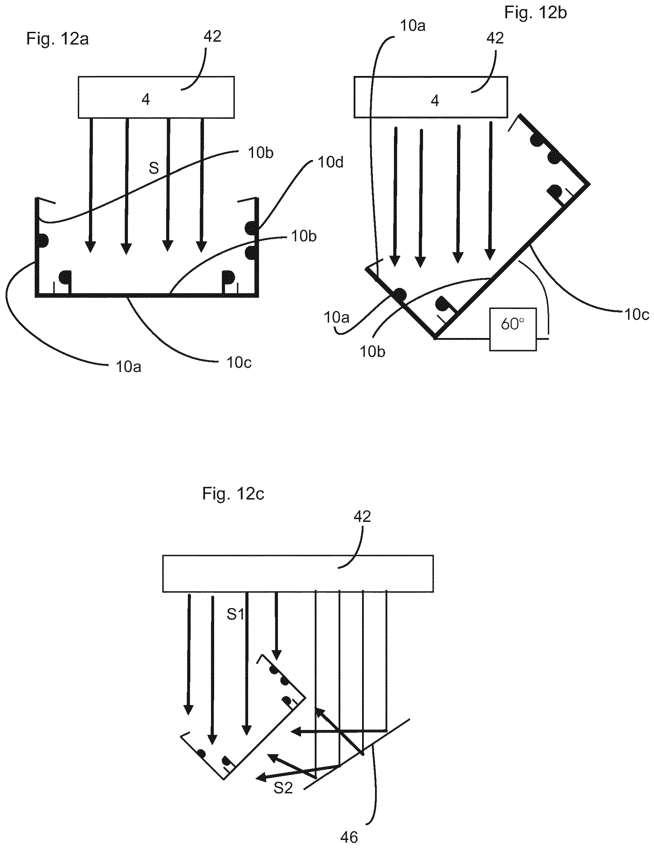

Die Fig. 12a bis 12c zeigen noch einmal schematische Darstellungen zur Veranschaulichung der Bestrahlung der Behältnisverschlüsse 10. Dabei bezieht sich das Bezugszeichen 10c auf eine untere äußere Bodenfläche des Behältnisverschlusses, das Bezugszeichen 10b auf eine innere Bodenfläche des Verschlusses, das Bezugszeichen 10a auf eine Außenwandung des Behältnisverschlusses 10 und das Bezugszeichen 10d auf Gewindeabschnitte. Bei der in Fig. 12a gezeigten Ausführungsform findet eine vertikale Bestrahlung statt, bei der in Fig. 12b gezeigten Ausführungsform eine Bestrahlung unter 60°. Bei der in 12c gezeigten Ausführungsform sind zwei Strahlungsrichtungen vorgesehen.

### Bezugszeichenliste

- 1: Vorrichtung
- 2: Transporteinrichtung
- 4: Bestrahlungseinrichtrung
- 10: Behältnisverschluss
- 10a: Umfangswandung des Behältnisverschlusses 10
- 10b: Innenbereich, innere Bodenfläche des Behältnisverschlusses 10
- 10c: äußere Bodenfläche des Behältnisverschlusses 10
- 10d: Gewinde des Behältnisverschlusses 10
- 22, 23: Transportband
- 24: erste Kontaktiereinrichtung
- 25: Stützfläche
- 26: zweite Kontaktiereinrichtung
- 27: Riffelung
- 30: Reinraum
- 31: Gehäuse des Reinraums
- 42: erste Strahlungseinrichtung
- 44: zweite Strahlungseinrichtung
- 45: Austrittsfenster
- 46: Strahlungsumlenkeinrichtung, Reflektorelement
- 47, 48: Elektronenerzeugungseinrichtung
- 52: Eintaktrad
- 54: Schiene
- 62: Zuführeinrichtung
- 64: Abführeinrichtung
- 66: Halteeinrichtung
- 67: Transportfläche
- 72: Zuführband
- 74: Antriebszahnrad
- 76: Antriebseinrichtung
- 82: Antriebseinrichtung
- 84: Transportsteg
- D: Drehachse
- P: Transportpfad
- S1, S2: Strahlungsrichtungen

## Patentansprüche

1. Verschlusssterilisationsanordnung (1) für Behältnisverschlüsse (10), mit einer Transporteinrichtung (2), welche die Behältnisverschlüsse (10) vereinzelt entlang eines vorgegebenen Transportpfads (P) transportiert, mit einer Antriebseinrichtung (76) zum Antreiben der Transporteinrichtung (2), mit einer Bestrahlungseinrichtung (4), welche eine die Behältnisverschlüsse (10) sterilisierende Strahlung erzeugt und die Behältnisverschlüsse (10) mit dieser Strahlung während deren Transport mit der Transporteinrichtung (2) beaufschlagt, mit einem Reinraum (30), innerhalb dessen die Behältnisverschlüsse (10) wenigstens zeitweise während ihrer Sterilisation gefördert werden, wobei die Verschlusssterilisationsanordnung (1) eine Dreheinrichtung (22, 23, 24, 26) aufweist, welche die Behältnisverschlüsse wenigstens zeitweise während ihres Transports mit der Transporteinrichtung (2) um eine vorgegebene Drehachse (D) dreht, und die Bestrahlungseinrichtung (4) derart gegenüber dem Transportpfad (P) der Behältnisverschlüsse (10) angeordnet ist, dass die von der Bestrahlungseinrichtung ausgehende Strahlung sowohl auf eine äußere Umfangswandung (10a) der Behältnisverschlüsse (10) trifft als auch in einen Innenbereich (10b) der Behältnisverschlüsse (10), wobei die Bestrahlungseinrichtung (4) die Behältnisverschlüsse (10) aus wenigstens zwei unterschiedlichen Strahlungsrichtungen (S1, S2) bestrahlt, und wobei die Transporteinrichtung (2) die Behältnisverschlüsse (10) mittels einzelner Mitnehmer (26) entlang des Transportpfades transportiert, und wobei diese Mitnehmer (26) an einem linearen Transportmittel der Transporteinrichtung (2) angeordnet sind.

2. Verschlusssterilisationsanordnung (1) nach Anspruch 1,
**dadurch gekennzeichnet, dass**
die Bestrahlungseinrichtung (4) eine erste Strahlungseinrichtung (42) aufweist, welche Strahlung unter einer ersten vorgegebenen Richtung (S1) auf die Behältnisverschlüsse (10) richtet, und eine zweite Strahlungseinrichtung (44), welche Strahlung unter einer zweiten vorgegebenen Richtung (S2), welche sich von der ersten Richtung unterscheidet, auf die Behältnisverschlüsse (10) richtet.

3. Verschlusssterilisationsanordnung (1) nach wenigstens einem der vorangegangenen Ansprüche,
**dadurch gekennzeichnet, dass**
die Bestrahlungseinrichtung (4) eine erste Strahlungseinrichtung (42) aufweist, welche Strahlung unter einer ersten vorgegebenen Richtung auf die Behältnisverschlüsse richtet, und eine Strahlungsumlenkeinrichtung (46), welche einen Teil der von der ersten Strahlungseinrichtung (42) austretenden Strahlung auf die Behältnisverschlüsse (10) umlenkt.

4. Verschlusssterilisationseinrichtung (1) nach wenigstens einem der vorangegangenen Ansprüche,
**dadurch gekennzeichnet, dass**
die Transporteinrichtung (2) derart gestaltet ist, dass sie die Behältnisverschlüsse (10) wenigstens zeitweise während ihrer Sterilisation derart fördert, dass eine Symmetrieachse (D) der Behältnisverschlüsse (10) schräg bezüglich einer Einstrahlrichtung (S1, S2) der sterilisierenden Strahlung steht.

5. Verschlusssterilisationseinrichtung (1) nach wenigstens einem der vorangegangenen Ansprüche,
**dadurch gekennzeichnet, dass**
die Transporteinrichtung (2) Stützkörper aufweist, welche die Behältnisverschlüsse während ihres Transports wenigstens teilweise an ihren Bodenflächen (10c) abstützen.

6. Verschlusssterilisationseinrichtung (1) nach wenigstens einem der vorangegangenen Ansprüche,
**dadurch gekennzeichnet, dass**
die Transporteinrichtung (2) eine erste Kontaktiereinrichtung (24) aufweist, welche die Behältnisverschlüsse (10) während deren Transport an einem Bereich ihrer Außenwandung und insbesondere Umfangswandung (10a) kontaktiert.

7. Verschlusssterilsationseinrichtung (1) nach Anspruch 6,
**dadurch gekennzeichnet, dass**
die Transporteinrichtung (2) eine zweite Kontaktiereinrichtung (26) aufweist, welche die Behältnisverschlüsse (10) während deren Transport an einem Bereich ihrer Außenwandung und insbesondere ihrer Umfangswandung (10a) kontaktiert.

8. Verschlusssterilisationsanordnung (1) nach Anspruch 7,
**dadurch gekennzeichnet, dass**
sich ein Reibungskoeffizient zwischen der ersten Kontaktiereinrichtung (24) und der Außenwandung (10a) der Behältnisverschlüsse (10) von einem Reibungskoeffizienten zwischen der zweiten Kontaktiereinrichtung (26) und der Außenwandung (10a, 10c) der Behältnisverschlüsse (10) unterscheidet.

9. Verfahren zum Sterilisieren von Behältnisverschlüssen (10), wobei die Behältnisverschlüsse (10) mittels einer Transporteinrichtung (2) entlang eines vorgegebenen Transportpfades (P) derart transportiert werden, dass sie einander zumindest zeitweise während dieses Transports nicht gegenseitig berühren, wobei die Behältnisverschlüsse (10) wenigstens zeitweise während dieses Transports mittels wenigstens
einer Bestrahlungseinrichtung (4) mit einer sterilisierenden Bestrahlung bestrahlt werden,
**dadurch gekennzeichnet, dass**
die Behältnisverschlüsse (10) wenigstens zeitweise während ihrer Sterilisation um eine vorgegebene Drehachse gedreht werden und wobei sowohl eine Umfangswandung (10a) der Behältnisveschlüsse (10) als auch ein Innenbereich der Behältnisverschlüsse von der Bestrahlungseinrichtung (4) bestrahlt wird, und wobei die Transporteinrichtung (2) die Behältnisverschlüsse (10) mittels einzelner Mitnehmer entlang des Transportpfades transportiert, und wobei diese Mitnehmer an einem linearen Transportmittel der Transporteinrichtung (2) angeordnet sind.

10. Verfahren nach Anspruch 9,
**dadurch gekennzeichnet, dass**
auch ein Bodenbereich (10c) der Behältnisverschlüsse (10) wenigstens zeitweise bestrahlt wird.

## Claims

1. A closure sterilization arrangement (1) for container closures (10), with a conveying device (2) which conveys the container closures (10) separately along a pre-set conveying path (P), with a drive device (76) for driving the conveying device (2), with an irradiation device (4) which generates a radiation sterilizing the container closures (10) and which acts upon the container closures (10) with this radiation during the conveying thereof by the conveying device (2), with a clean room (30) inside which the container closures (10) are conveyed at least for a time during the sterilization thereof,
wherein
the closure sterilization arrangement (1) has a rotating device (22, 23, 24, 26) which rotates the container closures about a pre-set axis of rotation (D) at least for a time during their conveying by the conveying device (2), and the irradiation device (4) is arranged with respect to the conveying path (P) of the container closures (10) in such a way that the radiation issuing from the irradiation device strikes both an external peripheral wall (10a) of the container closures (10) and an internal region (10b) of the container closures (10), wherein the irradiation device (4) irradiates the container closures (10) from at least two different radiation directions (S1, S2) and wherein the conveying device (2) conveys the container closures (10) by means of single carriers (26) along the conveying path and wherein these carriers (26) are arranged on a linear conveying means of the conveying device (2).

2. A closure sterilization arrangement (1) according to claim 1,
**characterized in that**
the irradiation device (4) has a first irradiation device (42), which directs radiation onto the container closures (10) in a first pre-set direction (S1), and a second radiation device (44), which directs radiation onto the container closures (10) in a second pre-set direction (S2) which is different from the first direction.

3. A closure sterilization arrangement (1) according to at least one of the preceding claims,
**characterized in that**
the irradiation device (4) has a first radiation device (42), which directs radiation onto the container closures in a first pre-set direction, and a radiation redirecting device (46), which re-directs part of the radiation issuing from the first radiation device (42) onto the container closures (10).

4. A closure sterilization arrangement (1) according to at least one of the preceding claims,
**characterized in that**
the conveying device (2) is designed in such a way that it conveys the container closures (10) at least for a time during the sterilization thereof, in such a way that an axis of symmetry (D) of the container closures (10) is orientated obliquely with respect to an irradiation direction (S1, S2) of the sterilizing radiation.

5. A closure sterilization arrangement (1) according to at least one of the preceding claims,
**characterized in that**
the conveying device (2) has support members which support the container closures at least in part on the base areas (10c) thereof during their conveying.

6. A closure sterilization arrangement (1) according to at least one of the preceding claims,
**characterized in that**
the conveying device (2) has a first contacting device (24) which contacts the container closures (10) during the conveying thereof on a region of their outer wall and in particular their peripheral wall (10a).

7. A closure sterilization arrangement (1) according to claim 6,
**characterized in that**
the conveying device (2) has a second contacting device (26) which contacts the container closures (10) during the conveying thereof on a region of their outer wall and in particular their peripheral wall (10a).

8. A closure sterilization arrangement (1) according to claim 7,
**characterized in that**
a coefficient of friction between the first contacting device (24) and the outer wall (10a) of the container closures (10) is different from a coefficient of friction between the second contacting device (26) and the outer wall (10a, 10c) of the container closures (10).

9. A method of sterilizing container closures (10), wherein the container closures (10) are conveyed along a pre-set conveying path (P) by means of a conveying device (2) in such a way that they do not mutually touch one another at least for a time during this conveying, wherein the container closures (10) are irradiated with a sterilizing irradiation by means of at least one irradiation device (4) at least for a time during this conveying,
**characterized in that**
the container closures (10) are rotated about a pre-set axis of rotation at least for a time during the sterilization thereof and wherein both a peripheral wall (10a) of the container closures (10) and an internal region of the container closures are irradiated by the irradiation device (4) and wherein the conveying device (2) conveys the container closures (10) by means of single carriers along the conveying path and wherein these carriers are arranged on a linear conveying means of the conveying device (2).

10. A method according to claim 9,
**characterized in that**
a base area (10c) of the container closures (10) is also irradiated at least for a time.

## Revendications

1. Ensemble de stérilisation de fermetures (1) pour des fermetures de récipient (10), avec un dispositif de transport (2), qui transporte les fermetures de récipient (10) de manière séparée le long d'un trajet de transport (P) prédéfini, avec un dispositif d'entraînement (76) servant à entraîner le dispositif de transport (2), avec un dispositif d'irradiation (4), qui produit un rayonnement stérilisant les fermetures de récipient (10) et qui soumet les fermetures de récipient (10) à l'action de ce rayonnement au cours de leur transport avec le dispositif de transport (2), avec une salle blanche (30), à l'intérieur de laquelle les fermetures de récipient (10) sont refoulées au moins de manière temporaire au cours de leur stérilisation,
dans lequel
l'ensemble de stérilisation de fermetures (1) présente un dispositif de rotation (22, 23, 24, 26), qui fait tourner les fermetures de récipient au moins de manière temporaire, au cours de leur transport avec le dispositif de transport (2), autour d'un axe de rotation (D) prédéfini, et le dispositif d'irradiation (4) est disposé en regard du trajet de transport (P) des fermetures de récipient (10) de telle manière que le rayonnement partant du dispositif d'irradiation atteint aussi bien une paroi périphérique (10a) extérieure des fermetures de récipient (10) qu'une zone intérieure (10b) des fermetures de récipient (10), dans lequel le dispositif d'irradiation (4) irradie les fermetures de récipient (10) depuis au moins deux directions de rayonnement (S1, S2) différentes, et dans lequel le dispositif de transport (2) transporte les fermetures de récipient (10) au moyen de divers entraîneurs (26) le long du trajet de transport, et dans lequel lesdits entraîneurs (26) sont disposés au niveau d'un moyen de transport linéaire du dispositif de transport (2).

2. Ensemble de stérilisation de fermetures (1) selon la revendication 1,
**caractérisé en ce que**
le dispositif d'irradiation (4) présente un premier dispositif de rayonnement (42), le rayonnement pointant, selon une première direction (S1) prédéfinie, sur les fermetures de récipient (10), et un deuxième dispositif de rayonnement (44), le rayonnement pointant, selon une deuxième direction (S2) prédéfinie, qui est différente de la première direction, sur les fermetures de récipient (10).

3. Ensemble de stérilisation de fermetures (1) selon au moins l'une quelconque des revendications précédentes,
**caractérisé en ce que**
le dispositif d'irradiation (4) présente un premier dispositif de rayonnement (42), le rayonnement pointant, selon une première direction prédéfinie, sur les fermetures de récipient, et un dispositif de déviation de rayonnement (46), qui dévie une partie du rayonnement sortant sur premier dispositif de rayonnement (42) sur les fermetures de récipient (10).

4. Dispositif de stérilisation de fermetures (1) selon au moins l'une quelconque des revendications précédentes,
**caractérisé en ce que**
le dispositif de transport (2) est configuré de telle manière qu'il refoule les fermetures de récipient (10) au moins de manière temporaire au cours de leur stérilisation de telle manière qu'un axe de symétrie (D) des fermetures de récipient (10) est oblique par rapport à une direction de rayon incident (S1, S2) du rayonnement stérilisant.

5. Dispositif de stérilisation de fermetures (1) selon au moins l'une quelconque des revendications précédentes,
**caractérisé en ce que**
le dispositif de transport (2) présente des corps d'appui, qui soutiennent les fermetures de récipient au cours de leur transport au moins en partie au niveau de leurs surfaces de fond (10c).

6. Dispositif de stérilisation de fermetures (1) selon au moins l'une quelconque des revendications précédentes,
**caractérisé en ce que**
le dispositif de transport (2) présente un premier dispositif d'établissement de contact (24), qui établit un contact avec les fermetures de récipient (10) au cours de leur transport au niveau d'une zone de leur paroi extérieure et en particulier au niveau de leur paroi périphérique (10a).

7. Dispositif de stérilisation de fermetures (1) selon la revendication 6,
**caractérisé en ce que**
le dispositif de transport (2) présente un deuxième dispositif d'établissement de contact (26), qui établit un contact avec les fermetures de récipient (10) au cours de leur transport au niveau d'une zone de leur paroi extérieure et en particulier de leur paroi périphérique (10a).

8. Dispositif de stérilisation de fermetures (1) selon la revendication 7,
**caractérisé en ce que**
un coefficient de frottement entre le premier dispositif d'établissement de contact (24) et la paroi extérieure (10a) des fermetures de récipient (10) est différent d'un coefficient de frottement entre le deuxième dispositif d'établissement de contact (26) et la paroi extérieure (10a, 10c) des fermetures de récipient (10).

9. Procédé servant à stériliser des fermetures de récipient (10), dans lequel les fermetures de récipient (10) sont transportées au moyen d'un dispositif de transport (2) le long d'un trajet de transport (P) prédéfini de telle manière qu'elles ne se touchent pas mutuellement au moins de manière temporaire au cours dudit transport, dans lequel les fermetures de récipient (10) sont irradiées d'une irradiation de stérilisation au moins de manière temporaire au cours dudit transport au moyen au moins d'un dispositif d'irradiation (4),
**caractérisé en ce que**
les fermetures de récipient (10) sont tournées au moins de manière temporaire au cours de leur stérilisation autour d'un axe de rotation prédéfini, et dans lequel aussi bien une paroi périphérique (10a) des fermetures de récipient (10) qu'une zone intérieure des fermetures de récipient sont irradiées par le dispositif d'irradiation (4), et dans lequel le dispositif de transport (2) transporte les fermetures de récipient (10) au moyen de divers entraîneurs le long du trajet de transport, et dans lequel lesdits entraîneurs sont disposés au niveau d'un moyen de transport linéaire du dispositif de transport (2).

10. Procédé selon la revendication 9,
**caractérisé en ce que**
également une zone de fond (10c) des fermetures de récipient (10) est irradiée au moins de manière temporaire.
